# EUROPEAN PATENT APPLICATION

(11) **EP 4 464 330 A1**
(43) Date of publication of application: **20.11.2024**
(21) Application number: 23305788.4
(22) Date of filing: 17.05.2023
(51) Int. Cl.: A61K 38/17, A61P 25/00, A61P 25/22, A61P 25/28, A61K 45/06

(54) **HIP/PAP PROTEIN OR A DERIVATIVE THEREOF FOR TREATING A COGNITIVE DISORDER ASSOCIATED WITH ANXIETY DISORDER**

(71) Applicant: THE HEALTHY AGING COMPANY, 75001 Paris (FR); University of South Florida, Tampa, FL 33620 (US)
(72) Inventor: MAGNAN, Christophe, 75020 Paris (FR); CRUCIANI-GUGLIELMACCI, Céline, 78360 MONTESSON (FR); AMOUYAL, Gilles, 75016 Paris (FR); BRECHOT, Christian, St Pete Beach 33706, FI (US); AMOUYAL, Paul, 75016 PARIS (FR); NASH, Kevin, Temple Terrace - FL 33617 (US); AMADO, Aurélie Joly, 34684 Palm Harbor (US)
(74) Representative: Cabinet Nony

(57) **Abstract**

The present invention relates to the use of the HIP/PAP protein, or a derivative thereof, in the treatment and prevention, and in particular in the treatment, of a cognitive disorder associated with anxiety disorder(s) in an individual in need thereof, as well as for improving cognition in an individual affected by a neurological disorder associated with anxiety disorder(s) or for alleviating cognitive deficit in an individual affected by a disorder selected from the group consisting of Obsessive-compulsive disorder, Attention deficit disorder, Dementia with Lewy bodies disease, Early onset dementia, Epilepsy-related cognitive dysfunction, Fronto-temporal dementia, Posterior cortical atrophy, Huntington's disease (HD), Parkinson's disease, bipolar disorder, substance abuse, attention deficit disorders, psychotic disorders and a sars-cov-2 infection.

## Description

### Field of the invention

The present invention relates to the use of the HIP/PAP protein, or a derivative thereof, in the treatment and prevention, and in particular in the treatment, of a cognitive disorder associated with anxiety disorder(s) in an individual in need thereof, as well as for improving cognition in an individual affected by a neurological disorder associated with anxiety disorder(s) or for alleviating cognitive deficit in an individual affected by particular cognitive disorders associated with anxiety disorder(s) as described below.

### Prior art

While occasional anxiety is a normal part of life, anxiety disorders involve more than temporary worry or fear. For people with an anxiety disorder, the anxiety does not go away and can get worse over time with symptoms interfering with daily activities such as job performance, schoolwork, and relationships. These symptoms need to be distinguished from normal anxiety and fear, which are healthy emotional reactions to daily stressors related to interpersonal, social, educational, and vocational demands. Anxiety disorders can manifest as an increased anxiety levels (feeling nervous, restless or tense, having a sense of impending danger, panic or doom, having difficulty controlling worry, having difficulty taking risks or decisions) or decreased anxiety levels also know as behavioral disinhibition and externalizing disorders (have a tendency to show high approach, absence of restraint, inappropriate laughter, disinhibition of speech and action in situations of novelty, high novelty seeking, and low harm avoidance high novelty - and sensation-seeking personality, poor impulse control, and proclivity to engage in risky behaviors - N. R. Marmorstein, J Anxiety Disord. 2007;21(3):420-32; J T Nigg, Psychol Bull. 2000 Mar;126(2):220-46; Dina R Hirshfeld-Becker et al., Biol Psychiatry. 2003 Jun 1;53(11):985-99) or a combination of both (N. R. Marmorstein, J Anxiety Disord. 2007;21(3):420-32; Audra K Langley et al., Eur Child Adolesc Psychiatry. 2010 Aug;19(8):637-45; B Wanner et al., Psychol Med. 2012 Nov;42(11):2373-82; Joan P Yoo et al., Am J Orthopsychiatry. 2009 Oct;79(4):532-40), especially in young adults. In certain instances, behavioral dishinibition can be a precursor of anxiety disorder showing the intricate relationship of both type of disorders (Dina R Hirshfeld-Becker et al., Biol Psychiatry. 2003 Jun 1;53(11):985-99).

Mood and anxiety disorders are characterized by a variety of neuroendocrine, neurotransmitter, and neuroanatomical disruptions. Identifying the most functionally relevant differences is complicated by the high degree of interconnectivity between neurotransmitter- and neuropeptide-containing circuits in limbic, brain stem, and higher cortical brain areas. Therefore, both high anxiety levels and low anxiety levels could share the same mechanism i.e imbalance of neurotransmitter signaling (Elizabeth I Martin et al., Psychiatr Clin North Am. 2009 Sep;32(3):549-75).

In the Region of the Americas, it was estimated in 2015 that more than 58 million people were suffering from anxiety disorders representing 7.7% in the female population, and 3.6% of the male population. Anxiety disorders are actually more prevalent than any other mental health disorder, composing the majority of lifetime mental health disorders worldwide (Kessler et al., Epidemiol Psichiatr Soc . 2009 Jan-Mar;18(1):23-33).

Anxiety disorders (including externalizing disorders) are often associated with cognitive disorders, either as a primary or as a secondary symptom.

An example of a cognitive disorder in which anxiety disorders are primary symptoms is obsessive-compulsive disorder, featuring a pattern of unwanted thoughts and fears, obsessions, that lead to do repetitive behaviors or mental acts, or compulsions, meant to reduce anxiety related to the obsessions (Anu E Castaneda et al., J Affect Disord. 2008 Feb;106(1-2):1-27; Ashwini Vishwanathan, Indian J Psychol Med. 2022 Nov;44(6):558-566). Cognitive dysfunction (decreased learning and working memory) has also been evidenced in externalizing disorders and behavioral disinhibition disorders of anxiety disorders as seen in bipolar disorder, as well as substance and alcohol abuse (Michael J Endres et al., J Abnorm Psychol. 2011 May;120(2):336-51).

Anxiety symptoms may alternatively be secondary to other disorders, and in particular may be secondary in several cognitive disorders. In these cognitive disorders, the alteration of the cognitive functions, in particular when associated with a light to severe memory loss, leads to an alteration of the normal response to anxiogenic situations and thus to anxiety disorders.

Not all cognitive disorders are associated with anxiety disorders. For example, can be cited some levels of mild cognitive impairments, normal pressure hydrocephalus or neurological disorders related cognitive dysfunction.

Depending on the considered cognitive disorder, or even depending on the stage of the considered cognitive disorder, the alteration of the normal response to an anxiogenic situation can either materialize by an increase or a decrease in anxiety-like behaviors.

Both share a common mechanism which comprises an imbalance in neurotransmission in the areas that control mood and anxiety.

Therefore, there is an increasing need for novel actives able to prevent and/or treat a cognitive disorder associated with anxiety disorder(s) in an individual in need thereof.

Moreover, there is an escalating need for the prevention of an alteration of the emotional reactions of an individual to stressors, in particular in an individual affected by a cognitive disorder which is expected to induce the development of (an) anxiety disorder(s).

There is indeed a need for novel actives able to restore healthy emotional reactions to stressors, in particular in an individual affected by a cognitive disorder associated with an anxiety disorder.

There is also a need for novel actives able to restore a physiological level of anxiety in an individual, in particular in an individual affected by a cognitive disorder associated with an anxiety disorder.

There is also a need for novel actives able to improve the memory of an individual affected by a cognitive disorder associated with anxiety disorder(s).

There is accordingly an increasing need for novel actives able to improve cognition in an individual affected by a neurological disorder associated with anxiety disorder(s), selected from the group consisting of Autism spectrum disorder, Angelman syndrome, Down's syndrome and other neurologic disorder-related cognitive impairments, such as Chronic meningitis, autoimmune encephalitis or neurosarcoidosis.

There is also a need for novel actives able to alleviate cognitive deficit in an individual affected by a disorder selected from the group consisting of Obsessive-compulsive disorder, Attention deficit disorder, Dementia with Lewy bodies disease, Early onset dementia, Epilepsy-related cognitive dysfunction, Fronto-temporal dementia, Posterior cortical atrophy, Huntington's disease (HD), Parkinson's disease (PD), bipolar disorder, substance abuse, attention deficit disorders, psychotic disorders and a sars-cov-2 infection.

The present invention provides a solution to these problems.

### Summary of the invention

The applicant has demonstrated that, surprisingly, the HIP/PAP protein, or a derivative thereof, makes it possible to improve the memory in a mouse model of cognitive disorders associated with anxiety. The HIP/PAP protein, or a derivative thereof, is also able to rescue cognitive deficits in this model and more particularly to restore a normal anxiety like behavior in response to an anxiogenic situation.

Without wishing to be bound by any theory, the inventors hypothesize that HIP/PAP can restore neurotransmitter homeostasis by direct or indirect action (on gabaergic and glutamatergic systems). Indeed, the results of the enclosed examples indicate a restoration of physiological levels of anxiety (to the level of controls) in the mice treated with HIP/PAP.

The HIP/PAP protein may therefore advantageously be used for:
- treating and/or preventing, in particular treating, a cognitive disorder associated with anxiety disorder(s) in an individual in need thereof;
   in particular treating and/or preventing, in particular treating, a cognitive disorder associated with an externalizing disorder, the cognitive disorder being in particular selected from the group consisting of bipolar disorder, substance abuse, attention deficit disorders and psychotic disorders;
- improving cognition in an individual affected by a neurological disorder associated with anxiety disorder(s), more particularly selected from the group consisting of Autism spectrum disorder, Angelman syndrome, Down's syndrome and other neurologic disorder-related cognitive impairments, such as Chronic meningitis, autoimmune encephalitis or *neurosarcoidosis*; and
- alleviating cognitive deficit in an individual affected by a disorder selected from the group consisting of Obsessive-compulsive disorder, Attention deficit disorder, Dementia with Lewy bodies disease, Early onset dementia, Epilepsy-related cognitive dysfunction, Fronto-temporal dementia, Posterior cortical atrophy, Huntington's disease (HD), Parkinson's disease (PD), bipolar disorder, substance abuse, attention deficit disorders, psychotic disorders and a sars-cov-2 infection.

The present invention accordingly relates to the following items:
**Item 1:** A HIP/PAP protein, or a derivative thereof, for its use in treating and/or preventing, in particular treating, a cognitive disorder associated with anxiety disorder(s) in an individual in need thereof.
**Item 2:** The HIP/PAP protein, or a derivative thereof, for use according to item 1 in which the anxiety disorder(s) associated with the cognitive disorder is:
   (i) a primary symptom, such as in Obsessive-compulsive disorder, and/or
   (ii) a secondary symptom, such as in a neurodegenerative disease selected in the group consisting of Attention deficit disorder, Dementia with Lewy bodies disease, Early onset dementia, Epilepsy-related cognitive dysfunction, Fronto-temporal dementia, Posterior cortical atrophy, Huntington's disease (HD), Parkinson's disease, bipolar disorder, substance abuse, attention deficit disorders, psychotic disorders and a sars-cov-2 infection.
**Item 3:** A HIP/PAP protein, or a derivative thereof, for use in improving cognition in an individual affected by a neurological disorder associated with anxiety disorder(s), selected from the group consisting of Autism spectrum disorder, Angelman syndrome, Down's syndrome and other neurologic disorder-related cognitive impairments, such as Chronic meningitis, autoimmune encephalitis or neurosarcoidosis.
**Item 4:** A HIP/PAP protein, or a derivative thereof, for use in alleviating cognitive deficit in an individual affected by a disorder selected from the group consisting of Obsessive-compulsive disorder, Attention deficit disorder, Dementia with Lewy bodies disease, Early onset dementia, Epilepsy-related cognitive dysfunction, Fronto-temporal dementia, Posterior cortical atrophy, Huntington's disease (HD), Parkinson's disease, bipolar disorder, substance abuse, attention deficit disorders, psychotic disorders and a sars-cov-2 infection.
**Item 5:** The HIP/PAP protein, or a derivative thereof, for use according to any one of items 1 to 4, wherein the anxiety disorder is an externalizing disorder and, in particular, the cognitive disorder is selected from the group consisting of bipolar disorder, substance abuse, attention deficit disorders and psychotic disorders.
**Item 6:** The HIP/PAP protein, or a derivative thereof, for use according to any one of items 1 to 5, wherein the individual is a mammal, in particular a human being.
**Item 7:** The HIP/PAP protein, or a derivative thereof, for use according to any one of items 1 to 6, wherein the HIP/PAP protein comprises an amino acid sequence selected from the group consisting of sequences set forth as SEQ ID NO: 1, SEQ ID NO: 2, SEQ ID NO: 3 and SEQ ID NO: 4, and is in particular the sequence set forth as SEQ ID NO: 4.
**Item 8:** The HIP/PAP protein, or a derivative thereof, for use according to any one of items 1 to 7, wherein said derivative comprises an amino acid sequence having a sequence identity of at least 80% with the amino acid sequence selected from the group consisting of sequences SEQ ID NO: 1, SEQ ID NO: 2, SEQ ID NO: 3 and SEQ ID NO: 4 and a biological activity of the same nature as the amino acid sequence selected from the group consisting of sequences SEQ ID NO: 1, SEQ ID NO: 2, SEQ ID NO: 3 and SEQ ID NO: 4, and more particularly a sequence identity of at least 80% with the amino acid sequence set forth as SEQ ID NO: 4 and a biological activity of the same nature as the amino acid sequence set forth as SEQ ID NO: 4.
**Item 9:** The HIP/PAP protein, or a derivative thereof, for use according to any one of items 1 to 8, wherein the HIP/PAP protein, or a derivative thereof, is in a composition comprising a physiologically acceptable medium.
**Item 10:** The HIP/PAP protein, or a derivative thereof, for use according to item 9, wherein the composition is for oral, sublingual, subcutaneous, intramuscular, intravenous, topical, local, intratracheal, intranasal or rectal administration.
**Item 11:** The HIP/PAP protein, or a derivative thereof, for use according to item 9 or 10, wherein the composition is for oral, subcutaneous, intravenous, topical or local administration.
**Item 12:** The HIP/PAP protein, or a derivative thereof, for use according to any one of items 9 to 11, wherein the composition further comprises at least one agent known for being useful in the prevention and/or treatment of a cognitive deficit, such has for example an agent selected from the group consisting of Cholinesterase inhibitors, such as donepezil, rivastigmine or galantamine; Glutamate regulators; such as memantine; cholinesterase inhibitors, in particular in combination with glutamate regulators, such as the combination of donepezil and memantine; Methylphenidate; Amphetamine; Atomoxetine; AMPA-R agonists; "Alpha7 nicotinic agonists"; Guanfacine; Bupropion; Vortioxetine and D-cycloserine.
**Item 13:** The HIP/PAP protein, or a derivative thereof, for use according to any one of items 9 to 12, wherein the composition further comprises at least one agent known for being useful in the prevention and/or treatment of an anxiety disorder, in particular selected from the group consisting of selective serotonin reuptake inhibitors, serotonin-norepinephrine reuptake inhibitors, tricyclic antidepressants, calcium modulators, azapirone, reversible inhibitors of monoamine oxidase A, agomelatine, quetiapine and vortioxetine, and more particularly selected from the group consisting of citalopram, escitalopram, fluoxetine, fluvoxamine, paroxetine, sertraline, duloxetine, venlafaxine, clomipramine, pregabalin, buspirone, moclobemide, agomelatine, quetiapine and vortioxetine.
**Item 14:** The HIP/PAP protein, or a derivative thereof, for use according to item 13, wherein the cognitive disorder associated with anxiety disorder(s) is selected from the group consisting of Obsessive-compulsive disorder, Attention deficit disorder, Dementia with Lewy bodies disease, Early onset dementia, Epilepsy-related cognitive dysfunction, Fronto-temporal dementia, Posterior cortical atrophy, Huntington's disease (HD), Parkinson's disease, bipolar disorder, substance abuse, attention deficit disorders, psychotic disorders and a sars-cov-2 infection.
**Item 15:** The HIP/PAP protein, or a derivative thereof, for use according to any one of items 9 to 14, wherein the composition further comprises at least one agent known for being useful in alleviating the symptoms associated with a disorder selected from the group consisting of Autism spectrum disorder, Angelman syndrome, Down's syndrome and other neurologic disorder-related cognitive impairments, such as Chronic meningitis, autoimmune encephalitis or neurosarcoidosis, in particular selected from the group consisting of an anti-seizure medication; amino acid supplements; antifungals; corticosteroids, such as prednisone; and immunosuppressants, such as methotrexate or azathioprine.

### BRIEF DESCRIPTION OF THE FIGURES

Figure 1A represents the locomotor activity, in particular the distance travaled in Opend Field) of various groups of mice: from left to right on the abscissa: normal control mice (Ntg - n=10); APP/PS1 mice treated with an rAAV9-empty capside (Tg Veh - control group - n=8); or APP/PS1 mice treated with rAAV9-hHIP/PAP (Tg HIP/PAP - n=8).
   Ordinate: Total distance traveled (m)
Figure 1B represents the Time Immobile in Opend Field of various groups of mice: from left to right on the abscissa: normal control mice (Ntg - n=10); APP/PS1 mice treated with an rAAV9-empty capside (Tg Veh - control group - n=8); or APP/PS1 mice treated with rAAV9-hHIP/PAP (Tg HIP/PAP - n=8).
   Ordinate: Total time immobile (seconds)
Figure 2 represents the results of a Novel object recognition test performed on various groups of mice: from left to right on the abscissa:
   normal control mice (Ntg - n=10); APP/PS1 mice treated with an rAAV9-empty capside (Tg Veh - control group - n=8); or APP/PS1 mice treated with rAAV9-hHIP/PAP (Tg HIP/PAP - n=8).
   each group showing results in the presence of a familiar object (left) or of a novel object (right).
   Ordinate: Time with objects (seconds).
   Two way ANOVA was performed, followed by Tukey's Multiple Comparisons post hoc test, set at a significance of p < 0.05. Data were analyzed using GraphPad Prism version 9 (GraphPad Software, San Diego California UDA, www.Graphpad.com).

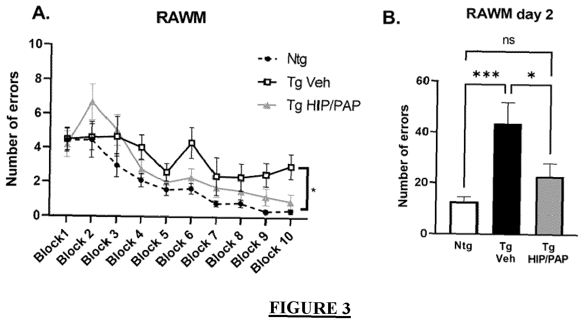

Figures 3A and 3B represent the results of a Radial arm water maze experiment performed on various groups of mice:
   normal control mice (Ntg - n=10); APP/PS1 mice treated with an rAAV9-empty capside (Tg Veh - control group - n=8); or APP/PS1 mice treated with rAAV9-hHIP/PAP (Tg HIP/PAP - n=8).
Figure 3A represents the number of errors for blocks of 3 consecutive trials averaged for each group of mice by the end of the test for a total of 15 trials during day 1 (blocks 1 to 5) and 15 trials during day 2 (blocks 6 to 10)(from left to right: Ntg; Tg Veh, Tg HIP/PAP).
   Ordinate: Number of errors
Figure 3B represents the sum of the total number of errors made by the mice in each group (Ntg 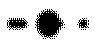 ; Tg Veh 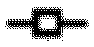 ; Tg HIP/PAP 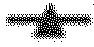 ) during day 2 (blocks 5 to 10).
   Ordinate: Number of errors
   For blocks of errors over the 2 days (Fig 3A), two-way ANOVA with repeated measures was performed, followed by Tukey's Multiple Comparisons post hoc test, set at a significance of p < 0.05. For total number of errors during day 2 (Fig 3B), one-way ANOVA was performed, followed by Tukey's Multiple Comparisons post hoc test, set at a significance of p < 0.05. Data were analyzed using GraphPad Prism version 9 (GraphPad Software, San Diego California UDA, www.Graphpad.com).

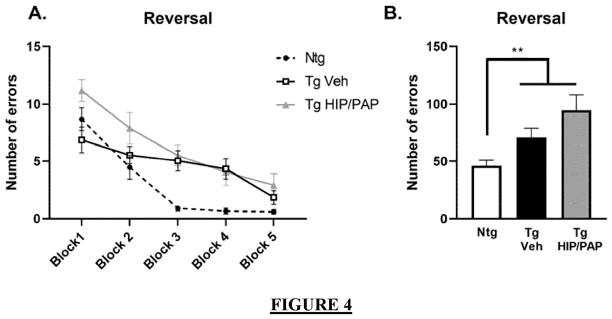

Figures 4A and 4B represent the results of a reversal trial of the Radial arm water maze experiment performed on various groups of mice: normal control mice (Ntg - n=10); APP/PS1 mice treated with an rAAV9-empty capside (Tg Veh - control group - n=8); or APP/PS1 mice treated with rAAV9-hHIP/PAP (Tg HIP/PAP - n=8).
Figure 4A represents the number of errors made by the mice of each group (Ntg 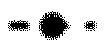 ; Tg Veh 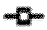 ; Tg HIP/PAP 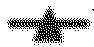 ) as blocks of 3 consecutive trials averaged for each group of mice by the end of the day for a total of 15 trials.
   Ordinate: block 1 to 5 (from left to right).
Figure 4B represents the combined number of errors made by each group of mice on all the trials (from left to right: Ntg; APPPS1 Empty; APPPS1 Reg 3).
   For blocks of errors (Fig 4A), two-way ANOVA with repeated measures was performed, followed by Tukey's Multiple Comparisons post hoc test, set at a significance of p < 0.05. For total number of errors (Fig 4B), one-way ANOVA was performed, followed by Tukey's Multiple Comparisons post hoc test, set at a significance of p < 0.05. Data were analyzed using GraphPad Prism version 9 (GraphPad Software, San Diego California UDA, www.Graphpad.com).

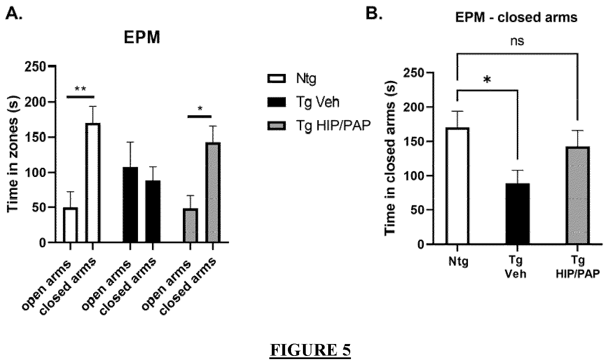

Figure 5 represents the anxiety like behavior of each group of mice detailed above evaluated by measurements of duration in open versus closed arms of an elevated plus maze (EPM), Fig 5 A, and a comparison of duration in closed arms only between the different groups (Fig5B).
   From left to right on the abscissa: normal control mice (Ntg - n=10); APP/PS1 mice treated with an rAAV9-empty capside (Tg Veh - control group - n=8); or APP/PS1 mice treated with rAAV9-hHIP/PAP (Tg HIP/PAP - n=8).
   Each group showing results in open arms (left) or closed arms (right).
   Ordinate: number of entries.

### Detailed description of the invention

### Definitions

In the context of the present invention, the terms "*prevent*", *"prevention"* and *"preventing"* denote the reduction to a lesser degree of the risk or of the probability of occurrence of a given phenomenon, that is to say, in the present invention, the prevention of a cognitive disorder associated with anxiety, in particular of complications selected from the group consisting of diabetic foot ulcer, foot infection and amputations. The terms "*prevent*", *"prevention"* and *"preventing"* also include preventing the aggravation, reducing the rate of progression or preventing recurrence of the said given phenomenon.

As used herein, the terms *"treating",* "*treatment*" or *"treat*" include alleviation of the symptoms associated with a specific disorder or condition and/or elimination of said symptoms, that is to say, in the present invention, the treatment of a cognitive disorder associated with anxiety.

A *"cognitive disorder"* is any disorder that significantly impairs the cognitive function of an individual to the point where normal functioning in society is impossible without treatment. Problems with a person's ability to think, learn, remember, use judgement, and make decisions. Cognitive disorder signs vary according to the particular disorder, but some common signs and symptoms overlap in most disorders. Some of the most common signs of cognitive disorder include: confusion, poor motor coordination, identity confusion, impaired judgment, memory loss (loss of short-term or long-term memory), trouble concentrating, completing tasks, understanding, remembering, following instructions, and solving problems. Other common signs may include changes in mood or behavior, loss of motivation, and being unaware of surroundings. Cognitive impairment may be mild or severe. Some cognitive disorders develop in stages and symptoms increase in severity the further the disease progresses. Cognitive instability comes with both short- and long-term effects. Some common short-term effects include ability to think and reason, memory loss, a state of confusion and a lack of coordination. Long-term effects include the increasing loss of declarative memory, such as forgetting names and significant faces, and a general lack of emotional stability and control over one's actions, as well as dementia (altered mental status and level of consciousness, shift in attention, mood swings, violent or unordinary behaviors, and hallucinations). According to the present invention, a cognitive disorder of interest is a cognitive disorder associated with anxiety disorder(s). By *"cognitive disorder associated with anxiety disorder(s)*", it is meant that the considered individual is affected by both at least one cognitive disorder and at least one anxiety disorder, said anxiety disorder being a primary or secondary symptom of the cognitive disorder. The terms *"primary symptom"* are used to describe a condition that's not caused by a different medical condition. The terms *"secondary symptom"* mean it is a consequence of another condition. Accordingly, according to the present invention, the terms *"cognitive disorder associated with anxiety disorder(s) "* include both the cognitive disorders which are a consequence of anxiety disorder(s) and cognitive disorders that led to the development of an anxiety disorder.

The diagnostic of cognitive disorders can be performed using different methods, amongst which, the Mini Mental Status Exam (MMSE), Montreal Cognitive Assessment (MoCA), Mini-Cog, and Cognitive Assessment Method (CAM), Glasgow Coma Score (GCS), Richmond Agitation and Sedation Scale (RASS) (Kelvin K F Tsoi et al., JAMA Intern Med. 2015 Sep; 175(9): 1450-8).

An *"anxiety disorder*" has been thoroughly defined above. Although feeling anxious from time to time is a common experience, anxiety disorders are different from typical or temporary feelings of worry or fear. Anxiety disorders can take different forms, including increased anxiety levels which can cause feelings of nervousness, restlessness, and impending danger, as well as difficulty controlling worry and making decisions. Alternatively, anxiety disorders can manifest as decreased anxiety levels, which can result in behavioral disinhibition and externalizing disorders. These disorders are characterized by a tendency to exhibit high approach, lack of restraint, inappropriate laughter, and disinhibition of speech and action in new situations. They may also involve high novelty-seeking, low harm avoidance, poor impulse control, and a proclivity for engaging in risky behaviors. In some cases, behavioral disinhibition may even be a precursor to anxiety disorders. Both mood and anxiety disorders are complex conditions involving disruptions to neuroendocrine, neurotransmitter, and neuroanatomical systems. Identifying the differences between these disorders can be difficult due to the complex interactions between different neural circuits in the brain. Nonetheless, both high and low anxiety levels may stem from a similar underlying cause, namely an imbalance in neurotransmitter signaling.

The diagnostic of anxiety disorder(s) can be performed using questionnaire such as the State-Trait Anxiety Inventory (STAI), the Generalized Anxiety Disorder 7 (GAD-7), the Beck Anxiety Inventory (BAI), the Zung Self-Rating Anxiety Scale, and the Taylor Manifest Anxiety Scale (Matthias Rose and Janine Devine, Dialogues Clin Neurosci. 2014 Jun;16(2):197-211). The diagnosis of anxiety disorders is made by symptoms, triggers, and a person's personal and family histories. There are no objective biomarkers or laboratory tests that can diagnose anxiety.

The terms "substance abuse" refer to the excessive and persistent use of a substance, such as alcohol or drugs, despite the negative consequences it may have on one's physical, psychological, or social well-being. Substance abuse can lead to addiction, which is a chronic and relapsing condition characterized by compulsive drug-seeking behavior and drug use despite harmful consequences. The diagnosis of substance abuse is usually based on criteria such as tolerance (the need for increased amounts of the substance to achieve the desired effect), withdrawal symptoms, continued use despite negative consequences, and unsuccessful attempts to quit or cut down use. It's worth noting that the definition of substance abuse may vary depending on the substance in question and the context in which it is used.

"Attention deficit disorders" (ADD), which comprises attention deficit hyperactivity disorder (ADHD), are medical conditions that are characterized by symptoms of inattention, hyperactivity, and impulsivity. Inattention symptoms may include difficulty paying attention, forgetfulness, poor organization, and distractibility. Hyperactivity symptoms may include fidgeting, restlessness, difficulty sitting still, and excessive talking. Impulsivity symptoms may include acting without thinking, interrupting others, and difficulty waiting for one's turn. The diagnosis of ADD/ADHD is usually based on a combination of symptoms, duration, and impairment in functioning, as well as ruling out other possible medical or psychological conditions that may be causing similar symptoms.

Psychotic disorders are a group of mental illnesses that are characterized by a loss of contact with reality, which may include symptoms such as delusions, hallucinations, disordered thinking, and abnormal behaviors. Delusions are fixed beliefs that are not based in reality and are often bizarre or paranoid in nature. Hallucinations are sensory experiences that are not based on external stimuli, such as hearing voices or seeing things that are not there. Disordered thinking may include difficulty with logical thinking, speech that is difficult to follow, and jumping between unrelated topics. Abnormal behaviors may include odd mannerisms or movements, lack of emotional expression, and social withdrawal.

Psychotic disorders can have a significant impact on a person's ability to function in daily life, and can range from mild to severe in severity. The most common psychotic disorders are schizophrenia, schizoaffective disorder, and delusional disorder.

"Cholinesterase inhibitors" are chemicals that prevent the breakdown of the neurotransmitter acetylcholine. Examples of cholinesterase inhibitors are donepezil, rivastigmine and galantamine. "Glutamate regulators" are chemicals that regulate the activity of the chemical messenger glutamate which is responsible for helping the brain process information. Such regulators are known for improving memory, attention, reason, language, and ability to perform simple tasks. An example of glutamate regulator is Memantine. "NMDA-R antagonists" are chemicals which can non-selectively decrease NMDA-Rs abnormal activity.

"Alpha7 nicotinic agonists" are chemicals which are implemented for the normalization of attentional dysfunctions. Examples fo such agonists can for example be clozapine or 3-2,4 dimethoxybenzylidene anabaseine (DMXBA). Other examples of Alpha7 nicotinic agonists are known, as illustrated for example in Laura F Martin et al., Psychopharmacology (Berl). 2004 Jun; 174(1):54-64 which is incorporated by reference.

"Selective serotonin reuptake inhibitors" are particularly known for being the most commonly prescribed antidepressants. Examples of such inhibitors can for example be citalopram, escitalopram, fluoxetine, fluvoxamine, paroxetine or sertraline.

"Serotonin-norepinephrine reuptake inhibitors" are a family of antidepressants that inhibit the reuptake of both serotonin and norepinephrine. Examples of such inhibitors are duloxetine and venlafaxine.

"Calcium modulator", also termed calcium channel modulators, of which the gabapentinoids are by far the most prominently prescribed, inhibit pain signals propagated by voltage-gated calcium channels situated at the central nerve terminal. Pregabalin may for example be cited as example of such modulator.

"Reversible inhibitors of monoamine oxidase A (RIMA)" inhibit the breakdown of three major neurotransmitters, serotonin, norepinephrine and dopamine, offering a multi-neurotransmitter strategy for in particular the treatment of depression. Moclobemide may for example be cited as example of such chemical.

"Anti-seizure medication" is a type of drug that is used to prevent or treat seizures or convulsions by controlling abnormal electrical activity in the brain. Anti-seizure medications are used to treat epilepsy and other seizure disorders. They are also used to treat medical conditions, such as bipolar disorder, nerve pain, migraine headaches, fibromyalgia, and restless leg syndrome.

"Antifungals" are medicines that kill or stop the growth of fungi (the plural of fungus) that cause infections. They are also called antimycotic agents.

"Corticosteroids", often known as steroids, are an anti-inflammatory medicine. As costicosteroid, Prednisone can for example be cited.

"Immunosuppressants", or immunosuppressive drugs, are key therapeutic tools that inhibit or prevent activity of the immune system. Can for example be cited as immunosuppressants methotrexate and azathioprine.

By "normal response to an anxiogenic situation", it is meant a physiological and psychological response that is appropriate and proportionate to the perceived level of threat or stress in an anxiogenic situation.

The terms "anxiogenic situation" refers to any situation or circumstance that is likely to provoke or increase anxiety in an individual. These situations may include, but are not limited to, public speaking, confrontations, social situations, uncertainty about the future, or situations that involve a potential threat to one's safety or well-being. Overall, anxiogenic situations are those that tend to elicit anxiety or fear in individuals.

The term *"physiologically acceptable medium"* is intended to denote a medium which is compatible with the body of the individual to whom said composition must be administered. It is, for example, a non-toxic solvent such as water. In particular, said medium is compatible with oral, sublingual, subcutaneous, intramuscular, intravenous, topical, local, intratracheal, intranasal or rectal administration, and more particularly with oral, subcutaneous, intravenous, topical or local administration.

The terms *"sequence homology"* or *"sequence identity"* or *"homology"* or *"identity"* are used interchangeably herein. For the purpose of the invention, it is defined here that in order to determine the percentage of sequence homology or sequence identity of two amino acid sequences or of two nucleic acid sequences, the sequences are aligned for optimal comparison purposes. In order to optimize the alignment between the two sequences gaps may be introduced in any of the two sequences that are compared. Such alignment can be carried out over the full length of the sequences being compared. Alternatively, the alignment may be carried out over a shorter length, for example over about 20, about 50, about 100 or more nucleic acids/based or amino acids. The sequence identity is the percentage of identical matches between the two sequences over the reported aligned region. A comparison of sequences and determination of percentage of sequence identity between two sequences can be accomplished using a mathematical algorithm. The skilled person will be aware of the fact that several different computer programs are available to align two sequences and determine the identity between two sequences (Kruskal, J. B. (1983) An overview of sequence comparison In D. Sankoff and J. B. Kruskal, (ed.), Time warps, string edits and macromolecules: the theory and practice of sequence comparison, pp. 1-44 Addison Wesley).

The percent sequence identity between two amino acid sequences or between two nucleotide sequences may be determined using the Needleman and Wunsch algorithm for the alignment of two sequences. (Needleman, S. B. and Wunsch, C. D. (1970) J. Mol. Biol. 48, 443-453). Both amino acid sequences and nucleotide sequences can be aligned by the algorithm. The Needleman-Wunsch algorithm has been implemented in the computer program NEEDLE.

For the purpose of the invention, the NEEDLE program from the EMBOSS package was used (version 2.8.0 or higher, EMBOSS: The European Molecular Biology Open Software Suite (2000) Rice, P. LongdenJ. And Bleasby,A. Trends in Genetics 16, (6) pp276-277, http://emboss.bioinformatics.nl/). For protein sequences EBLOSUM62 is used for the substitution matrix. For nucleotide sequence, EDNAFULL is used. The optional parameters used are a gap opening penalty of 10 and a gap extension penalty of 0.5. No end gap penalty is added. In the Output section, Yes has been indicated in response to the question "Brief identity and similarity" and "SRS pairwise" indicated as Output alignment format.

After alignment by the program NEEDLE as described above the percentage of sequence identity between a query sequence and a sequence of the invention is calculated as follows: Number of corresponding positions in the alignment showing an identical amino acid or identical nucleotide in both sequences divided by the total length of the alignment after subtraction of the total number of gaps in the alignment. The identity defined as herein can be obtained from NEEDLE by using the NOBRIEF option and is labeled in the output of the program as "longest-identity".

The similarity of nucleotide and amino acid sequences, i.e. the percentage of sequence identity, can be determined via sequence alignments using several other art-known algorithms, preferably with the mathematical algorithm of Karlin and Altschul (Karlin & Altschul (1993) Proc. Natl. Acad. Sci. USA 90: 5873-5877), with hmmalign (HMMER package, http://hmmer.wustl.edu/) or with the CLUSTAL algorithm (Thompson, J. D., Higgins, D. G. & Gibson, T. J. (1994) Nucleic Acids Res. 22, 4673-80) available e.g. on https://www.ebi.ac.uk/Tools/msa/clustalo/ or the GAP program (mathematical algorithm of the University of Iowa) or the mathematical algorithm of Myers and Miller (1989 - Cabios 4: 11-17) or Clone Manager 9. Preferred parameters used are the default parameters as they are set on https://www.ebi.ac.uk/Tools/msa/clustalo/.

The grade of sequence identity (sequence matching) may be calculated using e.g. BLAST, BLAT or BlastZ (or BlastX). A similar algorithm is incorporated into the BLASTN and BLASTP programs of Altschul et al (1990) J. Mol. Biol. 215, 403-410. BLAST polynucleotide searches are performed with the BLASTN program, score = 100, word length = 12, to obtain polynucleotide sequences that are homologous to those nucleic acids which encode the relevant protein.

BLAST protein searches are performed with the BLASTP program, score = 50, word length = 3, to obtain amino acid sequences homologous to the SHC polypeptide. To obtain gapped alignments for comparative purposes, Gapped BLAST is utilized as described in Altschul et al (1997) Nucleic Acids Res. 25, 3389-3402. When utilizing BLAST and Gapped BLAST programs, the default parameters of the respective programs are used. Sequence matching analysis may be supplemented by established homology mapping techniques like Shuffle-LAGAN (Brudno M., Bioinformatics 2003b, 19 Suppl 1: 154-162) or Markov random fields. When percentages of sequence identity are referred to in the present application, these percentages are calculated in relation to the full length of the longer sequence, if not specifically indicated otherwise.

In particular embodiments, % identity between two sequences is determined using CLUSTAL O (version 1.2.4).

As used herein, the term *"polypeptide"* refers to a molecule comprising amino acid residues linked by peptide bonds and containing more than five amino acid residues. The amino acids are identified by either the single-letter or three-letter designations. The term "protein" as used herein is synonymous with the term *"polypeptide"* and may also refer to two or more polypeptides. Thus, the terms *"protein", "peptide"* and *"polypeptide"* can be used interchangeably. Polypeptides may optionally be modified (e.g., glycosylated, phosphorylated, acylated, farnesylated, prenylated, sulfonated, and the like) to add functionality.

### HIP/PAP protein and derivative thereof implemented according to the invention

The HIP/PAP protein is known for its anti-apoptotic and mitogenic activity on hepatic cells (US 13/032,521, WO 2004/112824, Simon et al., FASEB J. 2003 Aug;17(11):1441-50).

It has also been shown that a 15-amino acid peptide, derived from the Reg IIIa family (HIP/PAP), the HIP (Human proIslet Peptide) peptide, has a regenerating activity on pancreatic islets and thus stimulates insulin production (US 2010/0093605).

The HIP/PAP protein according to the invention can comprise an amino acid sequence selected from the group consisting of sequences set forth as SEQ ID NO: 1, SEQ ID NO: 2, SEQ ID NO: 3 and SEQ ID NO: 4, in particular the sequence set forth as SEQ ID NO: 4.

The amino acid sequence SEQ ID NO: 1 corresponds to the HIP/PAP protein of sequence SEQ ID NO: 4, from which the N-terminal 26-amino acid signal peptide of said protein has been deleted.

In a particular embodiment, the HIP/PAP protein according to the invention comprises, or consists of, the amino acid sequence set forth as SEQ ID NO: 4.

In a particular embodiment, the HIP/PAP protein according to the invention comprises, or consists of, the amino acid sequence set forth as SEQ ID NO: 1.

The amino acid sequence SEQ ID NO: 2 corresponds to the short form of the HIP/PAP protein and, compared with the amino acid sequence SEQ ID NO: 1, has had the 11-amino acid propeptide in the N-terminal position deleted.

In a particular embodiment, the HIP/PAP protein according to the invention comprises, or consists of, the amino acid sequence set forth as SEQ ID NO: 2.

The sequence SEQ ID NO: 3 corresponds to the sequence SEQ ID NO: 1, to which a methionine has been added in the N-terminal position. The HIP/PAP derivative of sequence SEQ ID NO: 3 is also called rcHIP/PAP or ALF5755. This derivative may in particular be produced recombinantly in E.Coli cells. The 12-amino acid N-terminal propeptide (propeptide of 11 amino acids plus the additional methionine) may be cleaved, in order to obtain the short form of the HIP/PAP protein (SEQ ID NO: 2).

In a particular embodiment, the HIP/PAP protein according to the invention comprises, or consists of, the amino acid sequence set forth as SEQ ID NO: 3.

According to the invention, the short or long forms of the HIP/PAP protein or of the derivatives thereof may be used indifferently.

A derivative of the HIP/PAP protein according to the invention designates a biologically active derived form of a HIP/PAP protein of any one of sequences SEQ ID No. 1 to 4. The terms *"biologically active"* mean that the derivative of the HIP/PAP protein have the same biological activity as the HIP/PAP protein of any one of sequences SEQ ID No. 1 to 4.

A derivative of the HIP/PAP protein according to the invention comprises, or consists in, an amino acid sequence having a sequence identity of at least 80% with the amino acid sequence selected from the group consisting of sequences SEQ ID NO: 1, SEQ ID NO: 2, SEQ ID NO: 3 and SEQ ID NO: 4, in particular SEQ ID NO: 4, and a biological activity of the same nature as the amino acid sequence selected from the group consisting of sequences SEQ ID NO: 1, SEQ ID NO: 2, SEQ ID NO: 3 and SEQ ID NO: 4, in particular SEQ ID NO: 4.

A biological activity of the same nature as the HIP/PAP protein according to the present invention is as described previously, i.e. the capacity to treat and/or prevent peripheral neuropathy in an individual, in particular a diabetic peripheral neuropathy, and to accordingly prevent a complication of peripheral neuropathy in an individual, in particular a complication as detailed elsewhere in the present text.

As described herein, an amino acid sequence having at least 80% amino acid identity with a reference amino acid sequence encompasses amino acid sequences having at least 81%, 82%, 83%, 84%, 85%, 86%, 87%, 88%, 89%, 90%, 91%, 92%, 93%, 94%, 95%, 96%, 97%, 98% and 99% amino acid identity with the said reference amino acid sequence, and also a biological activity of the same nature as the said reference amino acid sequence.

In certain embodiments, the HIP/PAP protein, or a derivative thereof, may be associated or combined by noncovalent bonds with non-HIP/PAP portions. For example, the HIP/PAP protein or a derivative thereof could be associated with a liposome particle. Depending on the type of liposome, or on the production process, the HIP/PAP protein, or the derivative thereof, may be associated at the surface of the liposome or encapsulated inside said liposome.

The HIP/PAP protein, or a derivative thereof, may also be associated with non-HIP/PAP portions by covalent bonds. Such non-HIP/PAP portions may be selected from protein or non-protein compounds, for example polyethylene glycol, thus forming a pegylated HIP/PAP derivative.

A derivative of the HIP/PAP protein according to the invention also comprises the derivatives which become biologically active only when they are administered to the patient.

Finally, the derivatives of the HIP/PAP protein also comprise chimeric proteins or fusion proteins. Such proteins are fused with non-HIP/PAP polypeptides. The latter may be fused with the N- or C-terminal part. Typically, the HIP/PAP protein or a derivative thereof may be fused with the GST sequence at the level of their C-terminal part, in order to facilitate the purification of the recombinant proteins.

In certain embodiments of the invention, the HIP/PAP protein, or a derivative thereof, according to the invention is produced recombinantly in bacterial or animal cells, including insect and mammalian cells, according to techniques known to those skilled in the art.

In other embodiments, the HIP/PAP protein, or a derivative thereof, according to the invention may be isolated from a cell or from a tissue by known purification techniques.

The HIP/PAP protein and the derivatives thereof may also be produced by chemical synthesis.

In the text, the terms "*HIP*/*PAP protein"* cover the HIP/PAP protein in itself, and also the derivatives thereof as described above.

### Compositions implemented according to the invention

The invention also relates to the implementation of the HIP/PAP protein, or a derivative thereof, as defined previously in a composition comprising a physiologically acceptable medium.

The physiologically acceptable medium, previously defined in the present text, may be chosen, according to the pharmaceutical form and the mode of administration desired, from the usual excipients which are known to those skilled in the art (see Remington's Pharmaceutical Sciences, 16th edition, Osol, A ed., 1980).

By way of example, compositions implemented according to the invention may comprise, according to the therapeutic indications and the HIP/PAP protein or the derivative thereof:
a) the HIP/PAP protein or a derivative thereof; and
b) a buffer capable of maintaining the pH in a maximum stability range, preferentially from 1 to 9, more particularly from 4 to 8 and even more particularly from 6 to 7.5; and/or
c) a detergent or a surfactant which stabilizes the protein or the polypeptide against the aggregation induced by stirring; and/or
d) an isotonic; and/or
e) a preservative, chosen for example from the group consisting of phenols, benzyl alcohols, benzothelium halides, and chlorides; and/or
f) water.

If the detergent or the surfactant used is nonionic, it may be chosen from polysorbates, PLURONIC TM, polyethylene glycol (PEG) or poloxamers.

An isotonic will make it possible to maintain the isotonicity of the composition and will typically include polyalcohols such as glycerol, erythritol, arabitol, xylitol, sorbitol or mannitol, used alone or in combination. Alternatively, sodium chloride and/or any other inorganic salt may be used as isotonic.

The buffer may be, for example, acetate, citrate, succinate, a phosphate buffer or any other inorganic buffer, depending on the desired pH.

The preservatives of phenol, benzyl alcohol, benzothelium halide and chloride type are known antimicrobial agents. Typical preservatives comprise octadecyldimethylbenzylammonium chloride, hexamethonium chloride, benzalkonium chloride, phenol, butyl or benzyl alcohols, alkyl parabens, such as methyl or propyl paraben, catechol, resorcinol, cyclohexanol, 3-pentanol and m-cresol.

Additional excipients may also comprise antioxidants, such as ascorbic acid and methionine, chelating agents, such as EDTA, sugars, such as sucrose, mannitol, trehalose or sorbitol, etc.

The HIP/PAP protein, or the derivative thereof, according to the invention may be in the form of a pharmaceutically acceptable salt. This is intended to mean salts prepared from pharmaceutically acceptable non-toxic acids or pharmaceutically acceptable non-toxic bases, including organic and inorganic salts and acids. By way of example, mention may be made of alkali metal salts (sodium and potassium salts), alkaline-earth metal salts (calcium and magnesium salts), ammonium salts, salts of organic bases (pyridine or triethylamine salts), salts of inorganic acids (hydrochloride, sulfate, nitrate) and salts of organic acids (acetate, oxalate, p-toluenesulfonate).

A composition implemented according to the invention may be for oral, sublingual, subcutaneous, intramuscular, intravenous, topical, local, intratracheal, intranasal or rectal administration, and in particular for oral, subcutaneous, intravenous, topical or local administration.

According to one preferred embodiment, the HIP/PAP protein is administered in an effective amount, i.e. the amount required to obtain the expected effects of the invention. Such an amount of HIP/PAP protein will be determined, generally empirically, according to the subject to be treated and to his pathological condition. The effective amount will also depend on the mode of administration envisioned. The adjustments required to determine the effective amount for obtaining the maximum therapeutic effect correspond to techniques that are routine for the clinician.

An effective amount of the HIP/PAP protein or of a derivative thereof may for exemple be between 0.1 µg/day/kg of body weight and 100 mg/day/kg of body weight of the individual to which it must be administered. Although in certain embodiments an effective amount of the HIP/PAP protein, or derivative thereof, may reach more than 10 mg/kg, an effective amount of the HIP/PAP protein, or derivative thereof, according to the present invention is generally less than 5 mg/kg of body weight, which includes amounts less than 4.5 mg/kg, 4 mg/kg, 3.5 mg/kg, 3 mg/kg, 2.5 mg/kg or 2000 µg/kg. More particularly, an effective amount of the HIP/PAP protein, or derivative thereof, according to the present invention comprises amounts of at least 1 µg/kg, 2 µg/kg, 3 µg/kg, 4 µg/kg, 5 µg/kg, 6 µg/kg, 7 µg/kg, 8 µg/kg, 9 µg/kg, 10 µg/kg, 15 µg/kg, 20 µg/kg, 25 µg/kg, 30 µg/kg, 40 µg/kg, 50 µg/kg, 60 µg/kg, 70 µg/kg, 80 µg/kg, 90 µg/kg, 100 µg/kg, 150 µg/kg, 200 µg/kg, 250 µg/kg, 300 µg/kg, 350 µg/kg, 400 µg/kg, 450 µg/kg, 500 µg/kg, 600 µg/kg, 700 µg/kg, 800 µg/kg, 900 µg/kg, 1 mg/kg, 2 mg/kg, 3 mg/kg, 4 mg/kg, 5 mg/kg or more with respect to body weight of the individual to which it is or must be administered.

According to particular embodiments, the HIP/PAP protein, or derivative thereof, is administered according to a dosage of between 10 and 5000 µg/kg, preferentially between 100 and 2000 µg/kg with respect to body weight.

In the compositions implemented according to the present invention for oral, sublingual, subcutaneous, intramuscular, intravenous, topical, local, intratracheal or intranasal or rectal administration, the active ingredient (the HIP/PAP protein or a derivative thereof) may be administered in unit administration form as a mixture with pharmaceutical excipients.

When the composition is for oral administration, said composition may be chosen from the group consisting of a food product, a beverage, a pharmaceutical product, a nutraceutical, a food additive, a food supplement and a milk product.

The preferred modes of administration are the oral, subcutaneous, intravenous, topical or local routes, and more particularly the subcutaneous, intravenous, topical or local routes.

The administration of a compound or composition of the invention may for example be performed through the use of a sheath, a patch, a pad, a compress, a bandage, a tape, a gauze-based dressing, a woven or non-woven sponge or a syringe.

The HIP/PAP protein, or a derivative thereof, may be sterilized prior to the *in vivo* administration. The sterilization may be obtained by filtration on sterile filtration membranes, before or after the lyophilization or the reconstitution. The systemically administered HIP/PAP protein, or derivative thereof, may advantageously be lyophilized or stored in solution. In lyophilized form, the HIP/PAP protein, or derivative thereof, may be generally formulated in combination with excipients which enable reconstitution with an appropriate diluent, at the time of use.

The HIP/PAP protein, or a derivative thereof, may be administered daily in one intake or in a fractionated manner (for example from 2 to 3 times per day) until the desired therapeutic effect is obtained. It may also be administered chronically.

The HIP/PAP protein, or a derivative thereof, may also be administered in the form of a course, for example courses ranging from 15 days to 3 months, optionally repeated from 1 to 6 times at determined doses and time intervals.

The HIP/PAP protein, or a derivative thereof, according to the invention may also be combined in the context of a polytherapy with agents known for treating and/or preventing a cognitive deficit. Such additional agents known for being useful in the prevention and/or treatment of a cognitive deficit are known by the skilled person in the art and may in particular be selected from the group consisting of an agent selected from the group consisting of Cholinesterase inhibitors, such as donepezil, rivastigmine or galantamine; Glutamate regulators; such as memantine; cholinesterase inhibitors, in particular in combination with glutamate regulators, such as the combination of donepezil and memantine; Methylphenidate; Amphetamine; Atomoxetine; AMPA-R agonists; "Alpha7 nicotinic agonists"; Guanfacine; Bupropion; Vortioxetine and D-cycloserine.

The HIP/PAP protein, or a derivative thereof, according to the invention may also be combined in the context of a polytherapy with agents known for treating and/or preventing an anxiety disorder. Such additional agents known for being useful in the prevention and/or treatment of an anxiety disorder are known by the skilled person in the art and may in particular be selected from the group consisting of selective serotonin reuptake inhibitors, serotonin-norepinephrine reuptake inhibitors, tricyclic antidepressants, calcium modulators, azapirone, reversible inhibitors of monoamine oxidase A, agomelatine, quetiapine and vortioxetine, and more particularly selected from the group consisting of citalopram, escitalopram, fluoxetine, fluvoxamine, paroxetine, sertraline, duloxetine, venlafaxine, clomipramine, pregabalin, buspirone, moclobemide, agomelatine, quetiapine and vortioxetine.

A composition implemented according to the invention may accordingly comprise, in addition to the HIP/PAP protein, or a derivative thereof, according to the invention, at least an agent known for being useful in the prevention and/or treatment of a cognitive deficit and at least an agent known for being useful in the prevention and/or treatment of an anxiety disorder, which includes the case where one agent plays both roles, i.e. is known for being useful in the prevention and/or treatment of a cognitive deficit and of an anxiety disorder, such as votioxetine for example.

By administered in combination with, it means that the HIP/PAP protein, a derivative thereof, or a composition comprising it according to the invention can be administered simultaneously or sequentially compared to the other agents or compounds. When they are in separate compositions, the composition comprising the HIP/PAP protein, a derivative thereof, according to the invention and the composition comprising the at least one additional agent can be administered through the same route or through different routes.

The HIP/PAP protein, or a derivative thereof, according to the invention and the at least one additional agent can be administered in the same composition or in separate compositions.

By simultaneously, it is understood that the compositions can be administered at the same moment or up to the same day or couple of days.

By sequentially, it is understood that the compositions can be administered with at least several days, for example at least two days of difference.

### Implementation of the HIP/PAP protein and/or of a derivative thereof

As previously mentioned, a HIP/PAP protein, of a derivative thereof, as well as a composition comprising it, are for use in:
- the treatment and/or prevention, in particular the treatment, of a cognitive disorder associated with anxiety disorder(s) in an individual in need thereof; and/or
   in particular the treatment and/or prevention, in particular the treatment, of a cognitive disorder associated with an externalizing disorder, the cognitive disorder being in particular selected from the group consisting of bipolar disorder, substance abuse, attention deficit disorders and psychotic disorders; and/or
- restoring a physiological level of anxiety in an individual, in particular in an individual affected by a cognitive disorder associated with an anxiety disorder; and/or
- improving cognition in an individual affected by a neurological disorder associated with anxiety disorder(s), selected from the group consisting of Autism spectrum disorder, Angelman syndrome, Down's syndrome and other neurologic disorder-related cognitive impairments, such as Chronic meningitis, autoimmune encephalitis or *neurosarcoidosis*; and/or
- alleviating cognitive deficit in an individual affected by a disorder selected from the group consisting of Obsessive-compulsive disorder, Attention deficit disorder, Dementia with Lewy bodies disease, Early onset dementia, Epilepsy-related cognitive dysfunction, Fronto-temporal dementia, Posterior cortical atrophy, Huntington's disease (HD), Parkinson's disease (PD), bipolar disorder, substance abuse, attention deficit disorders, psychotic disorders and a sars-cov-2 infection.

As previously indicated, the anxiety disorder(s) associated with the cognitive disorder may in particular be:
(i) a primary symptom, such as in Obsessive-compulsive disorder, and/or
(ii) a secondary symptom, such as in a neurodegenerative disease selected in the group consisting of Attention deficit disorder, Dementia with Lewy bodies disease, Early onset dementia, Epilepsy-related cognitive dysfunction, Fronto-temporal dementia, Posterior cortical atrophy, Huntington's disease (HD), Parkinson's disease (PD), bipolar disorder, substance abuse, attention deficit disorders, psychotic disorders and a sars-cov-2 infection.

An individual to which the HIP/PAP protein, a derivative thereof, or a composition for use according to the invention, is administered may be a mammal and in particular a human being.

The HIP/PAP protein, a derivative thereof, or a composition for use according to the invention, may be administered in combination with standards of medical care for a cognitive deficit and/or for an anxiety disorder.

Accordingly, the HIP/PAP protein, a derivative thereof, or a composition for use according to the invention, may be administered to the individual in need thereof together with at least one agent known for being useful in the prevention and/or treatment of a cognitive deficit, such has for example an agent selected from the group consisting of Cholinesterase inhibitors, such as donepezil, rivastigmine or galantamine; Glutamate regulators; such as memantine; cholinesterase inhibitors, in particular in combination with glutamate regulators, such as the combination of donepezil and memantine; Methylphenidate; Amphetamine; Atomoxetine; AMPA-R agonists; "Alpha7 nicotinic agonists"; Guanfacine; Bupropion; Vortioxetine and D-cycloserine.

Alternatively, or additionnaly, the HIP/PAP protein, a derivative thereof, or a composition for use according to the invention, may be administered to the individual in need thereof together with at least one agent known for being useful in the prevention and/or treatment of an anxiety disorder, in particular selected from the group consisting of selective serotonin reuptake inhibitors, serotonin-norepinephrine reuptake inhibitors, tricyclic antidepressants, calcium modulators, azapirone, reversible inhibitors of monoamine oxidase A, agomelatine, quetiapine and vortioxetine, and more particularly selected from the group consisting of citalopram, escitalopram, fluoxetine, fluvoxamine, paroxetine, sertraline, duloxetine, venlafaxine, clomipramine, pregabalin, buspirone, moclobemide, agomelatine, quetiapine and vortioxetine.

When such an agent known for being useful in the prevention and/or treatment of an anxiety disorder is implemented with the HIP/PAP protein, a derivative thereof, or a composition for use according to the invention in a patient in need thereof, the cognitive disorder associated with anxiety disorder(s) may more particularly be selected from the group consisting of Obsessive-compulsive disorder, Attention deficit disorder, Dementia with Lewy bodies disease, Early onset dementia, Epilepsy-related cognitive dysfunction, Fronto-temporal dementia, Posterior cortical atrophy, Huntington's disease (HD), Parkinson's disease (PD), bipolar disorder, substance abuse, attention deficit disorders, psychotic disorders and a sars-cov-2 infection.

The HIP/PAP protein, a derivative thereof, or a composition for use according to the invention, may more particularly be administered together with at least one agent known for being useful in alleviating the symptoms associated with a disorder selected from the group consisting of Autism spectrum disorder, Angelman syndrome, Down's syndrome and other neurologic disorder-related cognitive impairments, such as Chronic meningitis, autoimmune encephalitis or neurosarcoidosis, in particular selected from the group consisting of an anti-seizure medication; amino acid supplements; antifungals; corticosteroids, such as prednisone; and immunosuppressants, such as methotrexate or azathioprine. In particular, a composition for use according to the invention may further comprise at least one agent known for being useful in alleviating the symptoms associated with a disorder selected from the group consisting of Autism spectrum disorder, Angelman syndrome, Down's syndrome and other neurologic disorder-related cognitive impairments, such as Chronic meningitis, autoimmune encephalitis or neurosarcoidosis, in particular selected from the group consisting of an anti-seizure medication; amino acid supplements; antifungals; corticosteroids, such as prednisone; and immunosuppressants, such as methotrexate or azathioprine.

The present invention also relates to a method for treating and/or preventing, in particular treating, a cognitive disorder associated with anxiety disorder(s) in an individual in need thereof, comprising administering to the said individual a HIP/PAP protein, a derivative thereof, or a composition comprising it.

The present invention also relates to a method for improving cognition in an individual affected by a neurological disorder associated with anxiety disorder(s), the neurological disorder associated with anxiety disorder(s) being selected from the group consisting of Autism spectrum disorder, Angelman syndrome, Down's syndrome and other neurologic disorder-related cognitive impairments, such as Chronic meningitis, autoimmune encephalitis or *neurosarcoidosis,* comprising administering to the said individual a HIP/PAP protein, a derivative thereof, or a composition comprising it.

The present invention also relates to a method for alleviating cognitive deficit in an individual affected by a disorder selected from the group consisting of Obsessive-compulsive disorder, Attention deficit disorder, Dementia with Lewy bodies disease, Early onset dementia, Epilepsy-related cognitive dysfunction, Fronto-temporal dementia, Posterior cortical atrophy, Huntington's disease (HD), Parkinson's disease, bipolar disorder, substance abuse, attention deficit disorders, psychotic disorders and a sars-cov-2 infection, comprising administering to the said individual a HIP/PAP protein, a derivative thereof, or a composition comprising it.

The present invention moreover relates to the use of a HIP/PAP protein, a derivative thereof, or a composition comprising it, for treating and/or preventing, in particular treating, a cognitive disorder associated with anxiety disorder(s) in an individual in need thereof.

The present invention moreover relates to the use of a HIP/PAP protein, a derivative thereof, or a composition comprising it, for improving cognition in an individual affected by a neurological disorder associated with anxiety disorder(s), the neurological disorder associated with anxiety disorder(s) being selected from the group consisting of Autism spectrum disorder, Angelman syndrome, Down's syndrome and other neurologic disorder-related cognitive impairments, such as Chronic meningitis, autoimmune encephalitis or *neurosarcoidosis.*

The present invention moreover relates to the use of a HIP/PAP protein, a derivative thereof, or a composition comprising it, for alleviating cognitive deficit in an individual affected by a disorder selected from the group consisting of Obsessive-compulsive disorder, Attention deficit disorder, Dementia with Lewy bodies disease, Early onset dementia, Epilepsy-related cognitive dysfunction, Fronto-temporal dementia, Posterior cortical atrophy, Huntington's disease (HD), Parkinson's disease (PD), bipolar disorder, substance abuse, attention deficit disorders, psychotic disorders and a sars-cov-2 infection.

The invention is described below in greater detail by means of the following examples which are given solely by way of illustration.

All the references to percentages are percentages by weight unless otherwise indicated.

### EXAMPLES

### Example 1

### Animal injections

Animals were anesthetized using isoflurane and positioned in a World Precision Instruments stereotactic surgery apparatus. An incision was made on the sagittal surface of the skull. Six months old APP/PS1 mice (denominated transgenic or Tg) were injected into the right and left hippocampus (HPC) (X=+/-2.7 Y=-2.7, Z=-3 from bregma) as well as right and left cortex (X=+/-2 Y=-2, Z=-3 from bregma) with 2 microliters of either rAAV9-empty capside (Transgenic vehicle, Tg Veh, n=8) or rAAV9-hHIP/PAP (Tg HIP/PAP, n=8) at 5×10¹¹µg/mL. Non-transgenic littermates (Ntg n=10) were used as controls for behavior baseline. Six months after surgery, animals underwent behavior testing for 2 weeks. At the end of behavior testing, animals were euthanized and tissue collected.

### Behavioral Analysis

Prior to euthanizing, mice were tested in a battery of behavioral tasks including open field, novel object recognition, elevated plus maze and radial arm water maze.. A group of age-matched wild type mice were used for comparison. Tasks were performed from least stressful (open field) to most stressful (RAWM) as described here-after.

Analysis of measures were performed using one-way or two-way ANOVA. Repeated measure was performed on tasks like water maze which require multiple training/trials. Tukey's HSD Post-hoc means comparisons for significant ANOVA measures were used using GraphPad Prism. Grubb's test was used to determine outliers.

### Open field

The open field was used as a standard test of general activity. Animals were monitored for 15 min in a 40 cm square open field with video tracking software (ANY-Maze, Stoelting, IL), under moderate lighting.

General activity levels were evaluated by measurements of horizontal and vertical activity.

### Novel object recognition test (NOR)

Mice were placed in a 40 × 40 cm arena monitored and quantified by video tracking (ANY-Maze, Stoelting, IL). Two objects similar in scale to the mouse were placed along the center line of the arena approximately 3-5 cm from the outside wall.

Each animal was given three acclimation trials of 5 minutes each with a 5 minutes inter-trial interval.

After each trial, the arena and object cues were cleaned with 70% ethanol to minimize olfactory cues. After the acclimation trials, one of the acclimated objects was replaced with a novel object. Animals were given a 5 minutes exploratory trial during which object exploration was monitored by video recording.

Working memory was evaluated by the time spent exploring both novel and familiar objects.

### Radial arm water maze and Reversal

The radial arm water maze contained 6 swim paths (arms) radiating from an open central area with a hidden escape platform located at the end of one of the arms. The pool was surrounded by several extra-maze cues to allow spatial navigation. On each trial, the mouse was allowed to swim for up to 60 seconds to find the escape platform. The platform was located in the same arm on each trial.

On day one, mice were given 15 trials alternating between a visible platform (above the water) and a hidden platform (below the water). On day two, mice were given 15 additional trials with all the trials using a hidden platform. The start arm was varied for each trial so that mice relied upon spatial cues to solve the task instead of learning motor rules (i.e. second arm on the right).

The goal arm for each mouse was different to avoid odor cues revealing the goal arm. Entry into an incorrect arm (all four limbs within the arm) was scored as an error. Failure to make an arm entry within 15 seconds was also scored as an error. The errors for blocks of 3 consecutive trials were averaged for data analysis. Mice averaging 1 error or less by the end of day 2 were considered to have reached the learning criterion.

On the third day, a reversal trial was performed with the goal platform placed in the arm 180° from the original location. Mice were given 15 trials, all with a hidden platform. Animals were monitored with video tracking software (Ethovision, Noldus).

### Elevated plus maze

The elevated plus maze test is one of the most widely used tests for measuring anxiety-like behavior.

The test is based on the natural aversion of mice for open and elevated areas, as well as on their natural spontaneous exploratory behavior in novel environments. Mice were placed at the junction of a four-arms maze composed of two open arms without walls and two arms enclosed by 15.25 cm high walls 30 cm long and 5 cm wide.

Animals were monitored for 5 min with video tracking software (ANY-Maze, Stoelting, IL), under moderate lighting. Anxiety like behavior was evaluated by measurements of number of entries/duration in open versus closed arms.

The more time the mice spend in the closed arms the more anxious they are, which is a normal behavior observed in the non-transgenic control mice.

### Results

### (i) Open field

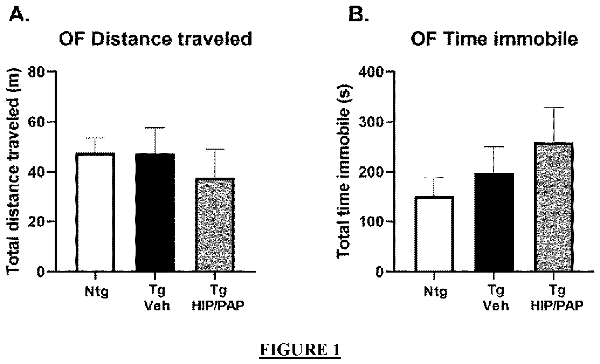

There was no difference in general activity between non-transgenic controls mice and transgenic mice, regardless of treatment. Overexpression of HIP/PAP in the brain of APP/PS1 mice did not induce any visible change in locomotor activity (see Figures 1A (Open Field - Distance traveled), 1B (Open Field - Time Immobile)

### (ii) Novel object recognition test (NOR)

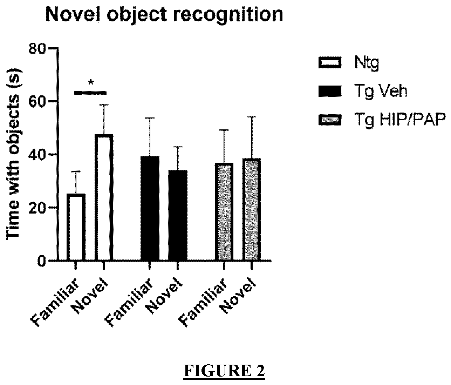

Upon exposure to the novel objet non-transgenic control mice spent significantly more time with the novel objet when compared to the familiar one, suggesting short memory of the previous trials. In contrast, Transgenic mice, regardless of treatment, spent an equal amount of time with both objects suggesting a deficiency in short memory. Overexpression of HIP/PAP in the brain of APP/PS1 mice did not rescue the genotype effect in short memory observed during novel object recognition (See Figure 2).

### (iii) Radial arm water maze

APP/PS1 mice treated with vehicle made significantly more errors in trying to reaching the platform than nontransgenic control mice, as evidenced by the higher number of errors in blocks (Fig 3A) and total errors during day 2 (Fig 3B). These results are indicative or cognitive dysfunction especially in learning and memory. APP/PS1 mice treated with HIP/PAP made significantly less errors than the APP/PS1 mice treated with vehicle indicating a rescue in cognitive function for this test (see Figures 3A and 3B).

### (iv) Reversal

As expected, APP/PS1 control mice made significantly more errors than non-transgenic littermates (see Figure 4B). There was no visible rescue in APP/PS1 mice treated with HIP/PAP (see Figure 4A).

### (v) Elevated plus maze

As shown in Figure 5A, non-transgenic control mice spent significantly more time in closed arms than open arms, exhibiting a normal amount of anxiety and fear of open space, a physiological defense mechanism. In contrast, vehicle treated transgenic mice spent the same amount of time in both arms, indicative of a deficit in anxiety-like behavior (Figure 5A). Moreover, when comparing the time spent in closed arms, vehicle treated transgenic mice spent significantly less time in closed arms compared to non-transgenic controls, indicative of behavior disinhibition and increased risk taking behavior (Figure 5B). Treatment with HIP/PAP in transgenic mice rescued a normal anxiety-like behavior as shown by the significant increase time spent in closed arms compared to open arms. Moreover, the increased time spend in closed arms observed upon treatment with HIP/PAP was significant against vehicle treated transgenic mice, but not non-transgenic mice, arguing in favor of a rescued phenotype, rather than an anxiogenic effect (Figure 5A).

### Conclusion

Overexpression of HIP/PAP in a transgenic mouse model significantly improved memory during Radial Arm Water Maze (RAWM) rescuing cognitive deficits during this test. A deficit in anxiety-like behavior was also rescued upon treatment with HIP/PAP during elevated plus maze when compared to transgenic vehicle treated mice.

HIP/PAP treatment accordingly allows the restoration in mice of a normal response to an anxiogenic situation. Treatment with HIP/PAP indeed normalized the anxiety like behavior that was lost in transgenic control mice.

### SEQUENCE LISTING

**SEQ ID NO: 1** is the amino acid sequence of the HIP/PAP protein from which the N-terminal 26-amino acid signal peptide has been deleted 
EEPQRELPSARIRCPKGSKAYGSHCYALFLSPKSWTDADLACQKRPSGNLVSVLSGAE
GSFVSSLVKSIGNSYSYVWIGLHDPTQGTEPNGEGWEWSSSDVMNYFAWERNPSTIS
SPGHCASLSRSTAFLRWKDYNCNVRLPYVCKFTD
**SEQ ID NO: 2** is the amino acid sequence of the HIP/PAP protein from which the N-terminal 26-amino acid signal peptide and the 11-amino acid propeptide in the N-terminal position have been deleted 
IRCPKGSKAYGSHCYALFLSPKSWTDADLACQKRPSGNLVSVLSGAEGSFVSSLVKSI
GNSYSYVWIGLHDPTQGTEPNGEGWEWSSSDVMNYFAWERNPSTISSPGHCASLSRS
TAFLRWKDYNCNVRLPYVCKFTD
**SEQ ID NO: 3** is the amino acid sequence of the HIP/PAP protein from which the N-terminal 26-amino acid signal peptide has been deleted and a methionine added in N-terminal position 
MEEPQRELPSARIRCPKGSKAYGSHCYALFLSPKSWTDADLACQKRPSGNLVSVLSG
AEGSFVSSLVKSIGNSYSYVWIGLHDPTQGTEPNGEGWEWSSSDVMNYFAWERNPST
ISSPGHCASLSRSTAFLRWKDYNCNVRLPYVCKFTD
**SEQ ID NO: 4** is the amino acid sequence of the full HIP/PAP protein

MLPPMALPSVSWMLLSCLMLLSQVQGEEPQRELPSARIRCPKGSKAYGSHCYALFLS
PKSWTDADLACQKRPSGNLVSVLSGAEGSFVSSLVKSIGNSYSYVWIGLHDPTQGTE
PNGEGWEWSSSDVMNYFAWERNPSTISSPGHCASLSRSTAFLRWKDYNCNVRLPYV
CKFTD

Amino acid sequences are disclosed in the present specification that serve as references. The same sequences are also presented in a sequence listing formatted according to standard requirements for the purpose of patent matters. In case of any sequence discrepancy with the standard sequence listing, the sequences described in the present specification shall be the reference.

## Claims

1. A HIP/PAP protein, or a derivative thereof, for its use in treating and/or preventing, in particular treating, a cognitive disorder associated with anxiety disorder(s) in an individual in need thereof.

2. The HIP/PAP protein, or a derivative thereof, for use according to claim 1 in which the anxiety disorder(s) associated with the cognitive disorder is:
(i) a primary symptom, such as in Obsessive-compulsive disorder, and/or
(ii) a secondary symptom, such as in a neurodegenerative disease selected in the group consisting of Attention deficit disorder, Dementia with Lewy bodies disease, Early onset dementia, Epilepsy-related cognitive dysfunction, Fronto-temporal dementia, Posterior cortical atrophy, Huntington's disease (HD), Parkinson's disease, bipolar disorder, substance abuse, attention deficit disorders, psychotic disorders and a sars-cov-2 infection.

3. A HIP/PAP protein, or a derivative thereof, for use in improving cognition in an individual affected by a neurological disorder associated with anxiety disorder(s), selected from the group consisting of Autism spectrum disorder, Angelman syndrome, Down's syndrome and other neurologic disorder-related cognitive impairments, such as Chronic meningitis, autoimmune encephalitis or *neurosarcoidosis.*

4. A HIP/PAP protein, or a derivative thereof, for use in alleviating cognitive deficit in an individual affected by a disorder selected from the group consisting of Obsessive-compulsive disorder, Attention deficit disorder, Dementia with Lewy bodies disease, Early onset dementia, Epilepsy-related cognitive dysfunction, Fronto-temporal dementia, Posterior cortical atrophy, Huntington's disease (HD), Parkinson's disease, bipolar disorder, substance abuse, attention deficit disorders, psychotic disorders and a sars-cov-2 infection.

5. A HIP/PAP protein, or a derivative thereof, for use according to any one of claims 1 to 4, wherein the anxiety disorder is an externalizing disorder and, in particular, the cognitive disorder is selected from the group consisting of bipolar disorder, substance abuse, attention deficit disorders and psychotic disorders.

6. The HIP/PAP protein, or a derivative thereof, for use according to any one of claims 1 to 5, wherein the individual is a mammal, in particular a human being.

7. The HIP/PAP protein, or a derivative thereof, for use according to any one of claims 1 to 6, wherein the HIP/PAP protein comprises an amino acid sequence selected from the group consisting of sequences set forth as SEQ ID NO: 1, SEQ ID NO: 2, SEQ ID NO: 3 and SEQ ID NO: 4, and is in particular the sequence set forth as SEQ ID NO: 4.

8. The HIP/PAP protein, or a derivative thereof, for use according to any one of claims 1 to 7, wherein said derivative comprises an amino acid sequence having a sequence identity of at least 80% with the amino acid sequence selected from the group consisting of sequences SEQ ID NO: 1, SEQ ID NO: 2, SEQ ID NO: 3 and SEQ ID NO: 4 and a biological activity of the same nature as the amino acid sequence selected from the group consisting of sequences SEQ ID NO: 1, SEQ ID NO: 2, SEQ ID NO: 3 and SEQ ID NO: 4, and more particularly a sequence identity of at least 80% with the amino acid sequence set forth as SEQ ID NO: 4 and a biological activity of the same nature as the amino acid sequence set forth as SEQ ID NO: 4.

9. The HIP/PAP protein, or a derivative thereof, for use according to any one of claims 1 to 8, wherein the HIP/PAP protein, or a derivative thereof, is in a composition comprising a physiologically acceptable medium.

10. The HIP/PAP protein, or a derivative thereof, for use according to claim 9, wherein the composition is for oral, sublingual, subcutaneous, intramuscular, intravenous, topical, local, intratracheal, intranasal or rectal administration.

11. The HIP/PAP protein, or a derivative thereof, for use according to claim 9 or 10, wherein the composition is for oral, subcutaneous, intravenous, topical or local administration.

12. The HIP/PAP protein, or a derivative thereof, for use according to any one of claims 9 to 11, wherein the composition further comprises at least one agent known for being useful in the prevention and/or treatment of a cognitive deficit, such has for example an agent selected from the group consisting of Cholinesterase inhibitors, such as donepezil, rivastigmine or galantamine; Glutamate regulators; such as memantine; cholinesterase inhibitors, in particular in combination with glutamate regulators, such as the combination of donepezil and memantine; Methylphenidate; Amphetamine; Atomoxetine; AMPA-R agonists; Alpha7 nicotinic agonists; Guanfacine; Bupropion; Vortioxetine and D-cycloserine.

13. The HIP/PAP protein, or a derivative thereof, for use according to any one of claims 9 to 12, wherein the composition further comprises at least one agent known for being useful in the prevention and/or treatment of an anxiety disorder, in particular selected from the group consisting of selective serotonin reuptake inhibitors, serotonin-norepinephrine reuptake inhibitors, tricyclic antidepressants, calcium modulators, azapirone, reversible inhibitors of monoamine oxidase A, agomelatine, quetiapine and vortioxetine, and more particularly selected from the group consisting of citalopram, escitalopram, fluoxetine, fluvoxamine, paroxetine, sertraline, duloxetine, venlafaxine, clomipramine, pregabalin, buspirone, moclobemide, agomelatine, quetiapine and vortioxetine.

14. The HIP/PAP protein, or a derivative thereof, for use according to claim 13, wherein the cognitive disorder associated with anxiety disorder(s) is selected from the group consisting of Obsessive-compulsive disorder, Attention deficit disorder, Dementia with Lewy bodies disease, Early onset dementia, Epilepsy-related cognitive dysfunction, Fronto-temporal dementia, Posterior cortical atrophy, Huntington's disease (HD), Parkinson's disease, bipolar disorder, substance abuse, attention deficit disorders, psychotic disorders and a sars-cov-2 infection.

15. The HIP/PAP protein, or a derivative thereof, for use according to any one of claims 9 to 14, wherein the composition further comprises at least one agent known for being useful in alleviating the symptoms associated with a disorder selected from the group consisting of Autism spectrum disorder, Angelman syndrome, Down's syndrome and other neurologic disorder-related cognitive impairments, such as Chronic meningitis, autoimmune encephalitis or neurosarcoidosis, in particular selected from the group consisting of an anti-seizure medication; amino acid supplements; antifungals; corticosteroids, such as prednisone; and immunosuppressants, such as methotrexate or azathioprine.
